# FASCICULE DE BREVET EUROPEEN

(11) **EP 1 397 429 B1**
(45) Date de publication et mention de la délivrance du brevet: **01.04.2015**
(21) Numéro de dépôt: 02732849.1
(22) Date de dépôt: 06.05.2002
(51) Int. Cl.: C08K 9/08, C08F 2/44

(54) **PARTICULES COMPOSITES, CONJUGUES DERIVES, PROCEDE DE PREPARATION ET APPLICATIONS**
VERBUNDTEILCHEN, KONJUGATE, VERFAHREN ZUR HERSTELLUNG UND VERWENDUNGEN
COMPOSITE PARTICLES, DERIVED CONJUGATES, PREPARATION METHOD THEREOF AND APPLICATIONS OF SAME

(30) Priorité: 10.05.2001 FR 0106203
(43) Date de publication de la demande: 17.03.2004
(73) Titulaire: BIO MERIEUX, 69280 Marcy l'Etoile (FR); CENTRE NATIONAL DE LA RECHERCHE SCIENTIFIQUE, 75794 Paris Cedex 16 (FR)
(72) Inventeur: ELAISSARI, Abdelhamid, 69230 saint Genis Laval (FR); MONTAGNE, Franck, F-69390 Charly (FR); BOSC, Eric, F-33770 Salles (FR); PICHOT, Christian, F-69960 Corbas (FR); MANDRAND, Bernard, F-69100 Villeurbanne (FR); BIBETTE, Jérôme, 75008 Paris (FR); MONDAIN-MONVAL, Olivier, 33400 Talence (FR)
(74) Mandataire: Guerre, Dominique
(86) Numéro de dépôt international: PCT/FR2002/001552
(87) Numéro de publication internationale: WO 2003/004559

(56) Documents cités:
- EP-A- 0 857 764
- FR-A- 2 503 728
- US-A- 5 814 687

## Description

La présente invention a pour objet des particules composites, leur procédé de préparation et leurs utilisations.

Les microsphères de type polymère présentent un intérêt comme support, vecteur ou véhicule dans les domaines de l'ingénierie biologique, du diagnostic et de la pharmacie. A cet effet, elles ont été utilisées dans le diagnostic médical comme support solide pour des molécules biologiques.

Les particules colloïdales présentent plusieurs avantages par rapport aux supports solides traditionnels, tels que tubes, plaques, billes, notamment parce qu'elles permettent de disposer d'une grande surface pour des interactions spécifiques et parce qu'elles sont facilement modifiables chimiquement pour introduire à leur surface des groupements fonctionnels susceptibles de réagir avec d'autres molécules, par exemple des molécules biologiques telles que des anticorps ou des fragments d'anticorps, des protéines, des polypeptides, des polynucléotides, des acides nucléiques, des fragments d'acides nucléiques, des enzymes ou des molécules chimiques telles que des catalyseurs, des médicaments, des molécules cages, des chélatants.

Parmi les particules colloïdales, les latex magnétiques ont suscité un grand intérêt dans le domaine analytique et sont utilisés par exemple comme moyen pour séparer et/ou détecter des analytes, tels que des antigènes, des anticorps, des molécules biochimiques, des acides nucléiques et autres.

Les particules composites de type polymère/magnétique sont habituellement classées en trois catégories sur un critère de taille : les petites particules ayant un diamètre inférieur à 50 nm, les grosses particules ayant un diamètre supérieur à 2 µm et les particules intermédiaires d'un diamètre compris entre 50 et 1000 nm.

Mais pour qu'elles puissent être considérées comme de bons candidats, en particulier pour une application diagnostique, elles doivent répondre à certains critères. D'un point de vue morphologique, il est préférable qu'elles soient relativement sphériques et que la charge magnétique soit répartie de façon relativement homogène dans la matrice polymère. Elles ne doivent pas s'agréger de manière irréversible sous l'action d'un champ magnétique, ce qui signifie qu'elles puissent être redispersées facilement, rapidement et de manière réversible. De même, elles doivent présenter une densité relativement faible pour réduire le phénomène de sédimentation. Avantageusement, elles doivent présenter une distribution granulométrique étroite. On parle encore de particules monodisperses ou isodisperses.

Ainsi, en raison de leur taille et de leur densité, les grosses particules magnétiques en suspension dans une phase liquide ont tendance à rapidement sédimenter. Par ailleurs, elles tendent à s'agréger après avoir été soumises à un champ magnétique car elles sont susceptibles d'avoir été de ce fait magnétisées de manière permanente (aimantation rémanente). Elles ne constituent donc pas un bon candidat.

A contrario, les petites particules magnétiques ont tendance à rester en suspension du fait de leur mouvement Brownien et sont difficilement attirées, voire pas du tout, par un aimant, en particulier si le champ magnétique appliqué est relativement faible. Elles ne sont donc pas bien appropriées pour les utilisations développées ci-dessus.

Il existe donc un intérêt évident à produire des particules composites de type polymère/magnétique, présentant une taille intermédiaire entre 50 et 1000 nm, qui à la fois pallient les inconvénients précités et répondent aux critères établis ci-dessus. Mais l'invention n'est pas limitée à des particules composites magnétisables, comme décrit ci après.

La demande de brevet EP-A-0 390 634 décrit des microsphères composites magnétisables de polymère vinylaromatique réticulé hydrophobe d'un diamètre de l'ordre de 50 à 10 000 nm et comprenant un coeur solide constitué de particules magnétisables et d'une écorce constituée d'un copolymère hydrophobe dérivé d'au moins un monomère vinylaromatique hydrophobe et d'au moins un polymère émulsifiant polyéthyléniquement insaturé soluble dans le ou les monomères vinylaromatiques et susceptible de réticuler avec le ou lesdits monomères. Toutefois, bien qu'elles puissent répondre à l'exigence de la taille, elles présentent l'inconvénient de ne pas avoir une répartition homogène de la charge magnétique qui est localisée à l'intérieur du coeur. Par ailleurs, et comme cela ressort à l'évidence des figures annexées, les particules ne sont pas homogène en taille. Il s'agit donc d'un ensemble de particules polydisperses qui devront être triées par application d'un champ magnétique pour ne retenir que les particules de taille attendue.

On peut également citer les particules Dynal (nom commercial). Ces particules sont des microsphères constituées d'un coeur poreux de polystyrène et d'oxydes de fer, les oxydes de fer ayant été déposés par imprégnation au niveau des pores disponibles à la surface du polystyrène, et d'une enveloppe en un autre polymère qui encapsule les oxydes de fer des microsphères poreuses. Elles présentent un diamètre respectivement de 2,8 µm (particules M280) et de 4,5 µm (particules M450) et sont relativement uniformes en taille. Elles sont donc considérées comme des particules isodisperses mais en raison de leur taille élevée présentent les inconvénients précités, principalement le phénomène de sédimentation. De plus, leur surface spécifique est faible.

Le document US-A-4 358 388 décrit des particules composites constituées par une matrice polymère dans laquelle une charge magnétique est répartie de manière homogène. Elles sont obtenues par polymérisation d'un monomère organique insoluble dans l'eau, dans une phase organique contenant la charge magnétique, ladite phase organique étant en émulsion dans une phase aqueuse en présence d'un agent tensioactif.

De telles particules constituent en biologie des supports similaires à ceux évoqués précédemment du type tubes, plaques ou billes, avec un atout supplémentaire résidant dans leur capacité à être isolées facilement par l'application d'un champ magnétique.

Néanmoins, leur utilisation dans ce domaine d'application reste assez limitée car ces particules ne présentent aucune fonctionnalisation.

Selon l'invention on dispose maintenant de nouvelles particules composites qui apportent une solution aux problèmes posés par les particules connues précédemment décrites et qui répondent aux critères suivants :
- Particules sphériques isodisperses,
- Particules possédant une charge inorganique répartie de manière homogène dans le coeur,
- Particules de taille intermédiaire, à savoir ayant un diamètre compris entre 50 et 1000 nm,
- Particules fonctionnalisées ou fonctionnalisables,
- La charge inorganique peut être magnétique ou magnétisable,
- Particules obtenues par un procédé de synthèse simple et contrôlable.

Les particules composites de l'invention sont des particules composites telles que revendiquées aux revendications 1 à 13 qui comprennent un coeur en un polymère hydrophobe et des nanoparticules inorganiques, ledit polymère hydrophobe constituant une matrice polymère à l'intérieur de laquelle les nanoparticules inorganiques sont stabilisées et distribuées de manière homogène, lesdites particules étant au moins partiellement entourées par un copolymère amphiphile comportant une partie hydrophobe et une partie hydrophile et dont la partie hydrophobe est au moins partiellement immobilisée sur ou dans ladite matrice polymère.

Selon une variante de l'invention, les particules sont complètement entourées par le copolymère amphiphile et/ou la totalité de la partie hydrophobe du copolymère amphiphile est immobilisée sur ou dans la matrice polymère.

On comprend selon l'invention un copolymère amphiphile, comme un copolymère possédant une partie hydrophobe et une partie hydrophile, étant entendu que la partie hydrophobe et/ou la partie hydrophile peuvent être un ensemble de sous-parties respectivement hydrophobes et/ou hydrophiles constitutives du copolymère amphiphile. Ainsi, un copolymère amphiphile de l'invention est notamment choisi parmi les copolymères en blocs, les copolymères séquencés, les polymères ramifiés dont le squelette est hydrophobe ou hydrophile et les ramifications sont respectivement hydrophiles ou hydrophobes, les polymères en peigne dont le squelette est hydrophobe ou hydrophile et les ramifications en peigne sont respectivement hydrophiles ou hydrophobes.

La partie hydrophile du copolymère amphiphile peut être ionique, anionique ou cationique, ou non ionique ; elle comporte des groupements fonctionnels qui sont réactifs, directement ou indirectement.

Selon l'invention, la partie hydrophobe est au moins partiellement immobilisée sur ou dans la matrice polymère. L'immobilisation de cette partie hydrophobe est toujours au moins de nature physique, et plus précisément mécanique, du type emprisonnement, et peut venir compléter une fixation préalable de la partie hydrophobe, de nature chimique ou physique comme une fixation par adsorption, par exemple. Ainsi, une immobilisation peut résulter des événements suivants :
- La partie hydrophobe du copolymère amphiphile est adsorbée à la surface de la matrice, puis définitivement immobilisée dans la matrice par le polymère hydrophobe ;
- Ladite partie hydrophobe est solubilisée dans la phase organique, puis emprisonnée dans le polymère hydrophobe lors de la formation de la matrice ;
- Ladite partie hydrophobe est uniquement emprisonnée mécaniquement lors de la formation de la matrice polymère.

Cette immobilisation conduit à des particules possédant une forte stabilité, et notamment bien supérieure à celle des particules connues décrites précédemment.

Un copolymère amphiphile approprié comprend avantageusement :
- Une partie hydrophobe choisie parmi les polystyrènes, les polyalkyles tels que le polyéthylène, les chaînes d'acides gras, et/ou
- Une partie hydrophile choisie parmi les polyacides acryliques, les polysulfates, les polyamines, les polyamides, les polysaccharides.

Les matériaux inorganiques constituant les nanoparticules sont choisis parmi les oxydes métalliques, tels que les oxydes de fer, de titane, de chrome, de cobalt, de zinc, de cuivre, de manganèse, de nickel et en particulier parmi les oxydes métalliques magnétisables, tels que les oxydes de fer, la magnétite, l'hématite ; les ferrites telles que les ferrites de manganèse, de nickel, de manganèse-zinc ; les alliages de cobalt, de nickel.

Avantageusement, les matériaux inorganiques sont choisis parmi les oxydes métalliques magnétisables, préférentiellement les oxydes de fer.

Les nanoparticules inorganiques représentent 5 à 95%, de préférence 10 à 90% et avantageusement 20 à 90% en masse par rapport à la masse totale de la particule composite, de préférence 25 à 85%.

A l'intérieur des particules de l'invention, les nanoparticules sont stabilisées par des agents de stabilisation choisis parmi les chaînes polymère amphiphiles, les agents tensioactifs ioniques ou non ioniques, fonctionnels ou non fonctionnels, polymérisables ou non polymérisables. Les agents tensioactifs fonctionnels sont en particulier choisis parmi les acides gras ou des dérivés d'acides gras, en particulier l'acide oléique ou ses dérivés, et les mélanges d'agents tensioactifs tels que définis précédemment.

Les polymères hydrophobes appropriés de la matrice sont les polymères de type vinylaromatique hydrophobe, c'est à dire les homopolymères de monomères vinylaromatiques insolubles dans l'eau, tels que styrène, méthylstyrène, éthylstyrène, tertio-butyl-styrène, vinyltoluène, ainsi que les copolymères de ces monomères entre eux et/ou avec d'autres comonomères, tels que les acrylates et les méthacrylates d'alkyle dans lesquels le groupement alkyle comprend de 3 à 10 atomes de carbone, les esters d'acides éthyléniques possédant 4 ou 5 atomes de carbone et d'alkyle possédant 1 à 8 atomes de carbone, les acides méthacryliques, les dérivés styréniques, les composés diéniques. Préférentiellement, le polymère est un polymère réticulé. Ceci est obtenu en ajoutant dans le ou les monomères une faible quantité (inférieure à 10% en poids) de molécules ayant au moins deux doubles liaisons réactives, telles que le divinyl benzène, le méthacrylate de vinyle, le cyanurate de triallyle, le diacrylate de mono ou de polyéthylèneglycol.

Les particules composites de l'invention peuvent également inclure un marqueur, tel qu'un marqueur radioactif, étant entendu que le marqueur est introduit lors de la préparation de l'émulsion telle que décrite dans l'exemple 1 qui suit.

Les particules de l'invention présentent un diamètre de l'ordre d'environ 50 à 1000 nm, de préférence d'environ 100 à 500 nm, et avantageusement d'environ 100 à 250 nm. Ce diamètre est défini à plus ou moins environ 5%, ce qui signifie que leur diamètre moyen en volume est défini à plus ou moins environ 5% près.

La présente invention a également pour objet un procédé de préparation des particules composites précitées qui permet d'obtenir directement des particules de diamètre souhaité et isodisperses sans nécessiter d'étape de tri en fonction du diamètre.

Selon un premier procédé de l'invention tel que revendiqué aux revendications 14 et 15 et 18 à 29, on obtient des particules composites pour lesquelles la partie hydrophobe du copolymère amphiphile est au moins partiellement immobilisée dans la matrice polymère, comme suit :
(i) on dispose d'une émulsion de départ stable et isodisperse constituée de deux phases non miscibles, une phase A hydrophobe constituée de gouttelettes contenant des nanoparticules inorganiques dispersées dans une phase organique contenant un agent tensioactif ; et une phase B hydrophile dans laquelle la phase A est dispersée ;
(ii) on introduit dans la phase B des monomères hydrophobes et au moins un agent amorceur ; et
(iii) on polymérise lesdits monomères hydrophobes à l'intérieur de la phase A,
et, selon ce procédé, on introduit en outre, dans la phase B, au moins un copolymère amphiphile, avant l'étape (iii), c'est-à-dire avant ou après l'étape (ii).

Selon un second procédé de l'invention tel que revendiqué aux revendications 16 à 29, on obtient des particules composites pour lesquelles la partie hydrophobe du copolymère amphiphile est au moins partiellement immobilisée sur la matrice polymère, comme suit :
(i) on dispose d'une émulsion de départ stable et isodisperse constituée de deux phases non miscibles, une phase A hydrophobe constituée de gouttelettes contenant des nanoparticules inorganiques dispersées dans une phase organique contenant un agent tensioactif ; et une phase B hydrophile dans laquelle la phase A est dispersée ;
(ii) on introduit dans la phase B des monomères hydrophobes et au moins un agent amorceur ;
(iii) on polymérise une fraction desdits monomères hydrophobes à l'intérieur de la phase A,
et, selon ce procédé, on introduit en outre, dans la phase B, au moins un copolymère amphiphile, avant l'étape (iii) ou après l'étape (iii), puis, lors d'une étape (iv), on polymérise la fraction restante des monomères hydrophobes à la surface de la matrice polymère. Quand le copolymère amphiphile est introduit avant l'étape (iii), il peut être introduit avant ou après l'étape (ii).

Pour obtenir les particules composites selon les procédés de l'invention, le polymère amphiphile est avantageusement choisi parmi ceux énoncés précédemment dans la description des particules composites.

Les monomères mis en oeuvre doivent former des polymères hydrophobes. Ils sont insolubles dans la phase hydrophile et choisis parmi les monomères vinylaromatiques tels que styrène, méthylstyrène, éthylstyrène, tertio-butyl-styrène, amino-méthylstyrène, vinyltoluène. Ils sont utilisés seuls ou en mélange ou bien encore en mélange avec d'autres monomères polymérisables insolubles dans l'eau tels que les acrylates, les méthacrylates d'alkyle, les esters d'acides éthyléniques et d'alkyle, les acides méthacryliques, les dérivés styréniques, les acides éthyléniques, les composés diéniques.

Il est possible d'ajouter au monomère ou au mélange de monomères un agent de réticulation hydrophobe, par exemple un monomère réticulant de type divinylbenzène, diméthacrylate, en particulier diméthacrylate de vinyle.

L'amorceur organosoluble est choisi parmi les amorceurs de type azobis, tels que les 2,2'-azobis(2,4-diméthyl valéronitrile), 2,2'-azobis(4-méthoxy-2,4-diméthylvaléronitrile), 2,2'-azobis(2-cyclopropylpropionitrile), 2,2'-azobis(2-méthylpropionitrile), 2,2'-azobis(2-méthylbutyronitrile), 1,1'-azobis(cyclohexane-1-carbonitrile), 1-((1-cyano-1-méthyléthyl)azo)formamide, 2-phénylazo-4-méthoxy-2,4-diméthyl-valéronitrile, diméthyl 2,2'-azobis(2-méthylpropionate, 4,4'-azobis(4-acide cyanovalérique) et 2,2'-azobis(2-(hydroxyméthyl)propionitrile). Si on choisit un amorceur hydrosoluble ou faiblement hydrosoluble, tels que les peroxydes, les hydroperoxydes et les persulfates, il engendre un début de polymérisation dans la phase hydrophile qui se propage dans la phase hydrophobe. Les persulfates, en particulier le persulfate d'ammonium, le persulfate de sodium et le persulfate de potassium sont solubles en phase aqueuse. Sous l'action de la chaleur, ils se décomposent et génèrent des anions à radicaux sulfate qui contribueront à charger la nanosphère composite. Le peroxyde d'hydrogène est soluble en phase aqueuse et génère des radicaux hydroxyles non chargés. La décomposition des hydroperoxydes génère un hydroxyle et un radical oxygéné qui se partageront dans une des phases en fonction de la nature du peroxyde utilisé. Ainsi, le peroxyde de cumène, dans le cas de la polymérisation du styrène, est supposé se décomposer au niveau de l'interface entre la particules de monomères et l'eau, les radicaux hydroxyles entrent dans la phase aqueuse et les radicaux non polaires diffusent vers la particule. De la nature cationique ou anionique de l'amorceur dépendra le caractère cationique ou anionique de la particule et du conjugué résultant de l'invention.

L'agent amorceur est introduit dans la phase hydrophile et pénètre dans la phase hydrophobe (a) soit simultanément à l'introduction des monomères hydrophobes, (b) soit préalablement à l'étape d'introduction des monomères hydrophobes, (c) soit postérieurement à l'étape d'introduction desdits monomères hydrophobes.

Dans un mode de réalisation préféré, l'étape (iii), et éventuellement l'étape (iv) de polymérisation sont effectuées par élévation de la température jusqu'à environ 60 à 90°C, en particulier à une température de 70°C, en présence de l'amorceur de polymérisation, étant entendu que les conditions de la polymérisation seront déterminées par l'homme du métier en fonction de la nature de l'amorceur retenu, ou par photochimie à l'aide de rayonnements, par exemple de rayonnements ultra violets ou d'un faisceau laser ou d'autres sources d'énergie.

La phase organique est une phase comprenant un hydrocarbure aliphatique ou cyclique choisi parmi les composés comprenant de 5 à 12 atomes de carbone, leurs isomères et leurs mélanges ou une phase comprenant tout ou partie d'un composé organique polymérisable par voie radicalaire. De préférence, l'hydrocarbure est choisi parmi le pentane, l'hexane, l'heptane, l'octane, le nonane, le décane, le undécane et le dodécane, et le composé organique polymérisable par voie radicalaire est choisi parmi les monomères vinylaromatiques insolubles dans l'eau, tels que styrène, méthylstyrène, éthylstyrène, tertio-butyl-styrène, vinyltoluène, ainsi que les copolymères de ces monomères entre eux, étant entendu qu'il est à la portée de l'homme du métier d'adapter les conditions de polymérisation en fonction du choix du ou des hydrocarbure(s) retenu(s) et de la nature de l'amorceur choisi. En particulier, si la polymérisation est effectuée par élévation de la température ou engendre une élévation de température, le montage réactionnel devra être adapté aux solvants volatils, tels que le pentane.

De manière préférentielle la phase hydrophile est une phase aqueuse, telle que de l'eau.

Dans des modes de réalisation préférentiels de la présente invention, la fontionnalisation des particules peut être complétée par introduction dans la phase B et pénétration dans la phase A de groupements réactifs fonctionnels. Les groupements réactifs fonctionnels sont apportés par exemple par des monomères faiblement hydrophiles susceptibles de polymériser avec les monomères hydrophobes de la matrice polymère. En particulier, les groupements réactifs fonctionnels sont apportés par des monomères hydrophiles choisis parmi les monomères des acides acryliques, méthacryliques, éthyléniques et sulfoniques, seuls ou en mélange, ou encore en mélange avec des monomères hydrophobes ; étant entendu qu'il est à la portée de l'homme du métier de déterminer la composition du mélange. Les groupements fonctionnels permettent les réactions ultérieures mais apportent également la stabilisation colloïdale nécessaire pour les applications ultérieures. Les groupements fonctionnels sont introduits dans la phase B et pénètrent à l'intérieur de la phase A (a) soit de manière simultanée à la pénétration des monomères hydrophobes de l'étape (ii), (b) soit préalablement à la pénétration des monomères hydrophobes de l'étape (ii), (c) soit postérieurement à la pénétration des monomères hydrophobes de l'étape (ii).

Dans un mode de réalisation particulier, et si souhaité, l'étape (iii) est suivie d'une étape d'évaporation partielle ou totale de la phase organique A avec formation de particules composites poreuses.

L'invention fournit donc des particules composites qui sont fonctionnalisées par la partie hydrophile du copolymère amphiphile, et qui peuvent au surplus l'être par la présence, de groupements fonctionnels réactifs, apportés comme indiqué ci-dessus au cours du procédé de préparation. Les particules de l'invention sont fonctionnalisées de préférence par des groupements choisis parmi les groupements carboxylique, amine, thiol, hydroxyl, tosyl hydrazine, qui seront à même de réagir avec au moins un ligand.

Les particules composites fonctionnalisées, non poreuses ou poreuses, ainsi formées seront susceptibles d'immobiliser un ligand, par exemple une molécule biologique, telle qu'un anticorps, un fragment d'anticorps, une protéine, un polypeptide, une enzyme, un polynucléotide, une sonde, une amorce, un fragment d'acide nucléique ; des molécules chimiques, telles que des polymères chimiques, des substances médicamenteuses, des molécules cages, des agents chélatants, des catalyseurs, la biotine ; étant entendu que lorsque les particules composites sont dites « poreuses », elles ont atteints un degré de porosité déterminé par évaporation totale ou partielle de la phase organique.

Aussi, la présente invention a également pour objet des conjugués constitués par des particules composites de l'invention telles que définies à la revendication 5, dont les groupements réactionnels ont réagi, directement ou indirectement, avec au moins un ligand tel que défini ci-dessus, ainsi que leurs utilisations.

A titre d'exemple, lesdits conjugués sont utilisés dans des tests immunologiques pour la détection et/ou la quantification de protéines, d'antigènes, d'anticorps dans un échantillon biologique ou dans des essais utilisant la technologie des sondes pour la détection et/ou la quantification d'un fragment d'acide nucléique dans un échantillon biologique. L'utilisation de sondes pour la détection et/ou la quantification d'un acide nucléique dans un échantillon est bien connue de l'homme du métier et on peut citer à titre d'illustration la technique d'hybridation sandwich. De même, les conjugués de l'invention peuvent être utilisés comme « agents porteurs d'amorces » pour une réaction d'amplification d'acides nucléiques dans un échantillon, par exemple par PCR (Polymerase Chain Reaction) ou toute autre technique d'amplification appropriée, permettant ainsi la détection et/ou la quantification d'acides nucléiques dans l'échantillon biologique.

La présente invention a donc également pour objet un réactif et une composition diagnostique comprenant en outre lesdits conjugués et l'utilisation dudit réactif dans un essai diagnostique.

Les conjugués trouvent également une application dans le domaine thérapeutique comme véhicule ou vecteur d'une substance médicamenteuse, d'un agent réparateur de gènes défectueux, d'un agent susceptible de bloquer l'expression d'un gène, tel qu'une sonde anti-sens en thérapie ou d'un agent susceptible de bloquer l'activité d'une protéine et de ce fait ils peuvent être utilisés dans une composition thérapeutique ou prophylactique.

Ainsi, les conjugués de l'invention sont susceptibles de véhiculer une substance médicamenteuse dans une composition thérapeutique ou prophylactique qui comprend ledit conjugué en association avec un adjuvant et/ou diluant et/ou excipient approprié et pharmaceutiquement acceptable, ladite substance médicamenteuse étant capable d'être relarguée *in vivo.* Les définitions des excipients et adjuvants pharmaceutiquement acceptables sont décrits par exemple dans Remington's Pharmaceutical Sciences 16th ed., Mack Publishing Co.

Les conjugués de l'invention sont également susceptibles de véhiculer un gène d'intérêt thérapeutique codant pour au moins une protéine d'intérêt ou un fragment d'une protéine d'intérêt, étant entendu que par protéine on entend à la fois une protéine dans sa définition la plus généralement utilisée et un anticorps. Bien entendu, un tel conjugué est incorporé dans une composition thérapeutique ou prophylactique qui comprend également les éléments nécessaires à l'expression dudit gène d'intérêt thérapeutique.

Les conjugués de l'invention sont également utilisables, quand incorporés dans une composition thérapeutique ou prophylactique, pour le transfert *in vivo* de sondes ou oligonucléotides anti-sens. Les anti-sens sont capables d'interférer spécifiquement avec la synthèse d'une protéine cible d'intérêt, par inhibition de la formation et/ou du fonctionnement du polysome selon le positionnement de l'ARNm dans la cible. Donc le choix fréquent de la séquence entourant le codon d'initiation de la traduction comme cible pour une inhibition par un oligonucléotide anti-sens vise à prévenir la formation du complexe d'initiation. D'autres mécanismes dans l'inhibition par des oligonucléotides anti-sens impliquent une activation de la ribonucléase H qui digère les hybrides oligonucléotide anti-sens/ARNm ou une interférence au niveau de sites d'épissage par des oligonucléotides anti-sens dont la cible est un site d'épissage de l'ARNm. Les oligonucléotides anti-sens sont également complémentaires de séquences ADN et peuvent donc interférer au niveau de la transcription par la formation d'une triple hélice, l'oligonucléotide anti-sens s'appariant par des liaisons hydrogène dites de Hoogsteen au niveau du grand sillon de la double hélice d'ADN. Dans ce cas particulier, on parle plus précisément d'oligonucléotides antigènes. Il est bien entendu que les oligonucléotides anti-sens peuvent être strictement complémentaires de la cible ADN ou ARN à laquelle ils doivent s'hybrider, mais aussi non strictement complémentaires à la condition qu'ils s'hybrident à la cible. De même, il peut s'agir d'oligonucléotides anti-sens non modifiés ou modifiés au niveau des liaisons inter-nucléotidiques. Toutes ces notions font partie des connaissances générales de l'homme de l'art.

La présente invention concerne donc une composition thérapeutique comprenant, entre autres, un conjugué vecteur d'un oligonucléotide anti-sens tel que définis ci-dessus.

Enfin, les conjugués sont susceptibles de former des complexes du type molécule cage/cryptate, chélatant/molécule chélatée ou de servir de véhicule pour des catalyseurs dans une application chimique.

Les particules composites de l'invention sont obtenues par polymérisation en émulsion *in situ* selon le protocole décrit dans les exemples qui suivent.

### Exemple 1 : préparation d'une émulsion stable et isodisperse

Une émulsion de départ stable et isodisperse a été préparée conformément à l'un ou l'autre des protocoles décrits dans cet exemple.
(i) L'émulsion primaire a été préparée à l'aide d'un procédé d'émulsification en incorporant progressivement, tout en cisaillant à l'aide d'un moulin colloïdal (Ika : nom commercial), la phase dispersée, formée de 45% en poids d'oxydes de fer dans de l'octane, à la phase continue formée de dodécyl sulfate de sodium à une concentration de 50% en poids dans l'eau jusqu'à l'obtention de fractions comprenant de 80% en poids de ferrofluide organique. Le mélange ainsi défini a été fragmenté dans une couette de type PG398 à un taux de cisaillement préalablement déterminé. L'émulsion primaire ainsi préparée est une émulsion polydisperse caractérisée par une distribution large du diamètre des gouttelettes qui est ensuite traitée par des tris magnétiques successifs pour l'obtention de l'émulsion de départ isodisperse en taille.
(ii) L'émulsion primaire a été préparée à l'aide d'un procédé d'émulsification en ajoutant rapidement la phase dispersée, formée d'octane, de 73% en poids d'oxydes de fer et d'un agent tensioactif lipophile de type monoglycérol ou polyglycérol de polyrisinoléate (1 à 10% en poids), à la phase continue formée de tensioactif de type tergitol NP10 (31% en poids) grâce à une spatule. Le mélange ainsi défini est ensuite fragmenté dans une couette de type PG398 à un taux de cisaillement préalablement défini. L'émulsion primaire ainsi préparée est une émulsion relativement isodisperse caractérisée par une distribution faible du diamètre des gouttelettes qui est ensuite traitée par des tris magnétiques successifs pour l'obtention de l'émulsion de départ isodisperse en taille.

### Exemple 2 : préparation de particules composites avec un copolymère amphiphile « en peigne »

L'émulsion préparée selon le protocole décrit dans l'Exemple 1 (i) (50 ml ; Ts = 5%) initialement stabilisée dans le Triton X405 (2 g/l) est lavée à trois reprises par séparation magnétique avec une solution de polymère (1,5 g/l ; pH8). A chaque lavage, le surnageant limpide est éliminé et remplacé par un volume égal de solution contenant le copolymère. Le copolymère amphiphile est un polyacide acrylique modifié par des chaînons hydrophobes (Mw = 20 000 g/mol). Ce polymère est composé par 90% de motifs acide acrylique et par 10% de motifs méthacrylate de polyoxyalkylène / aryle (Coatex JS3879B). Le copolymère possède des propriétés amphiphiles qui permettent de venir s'adsorber aux interfaces eau - huile. Apres avoir été filtrée sur un filtre nylon de 1 µm, l'émulsion est introduite dans un réacteur de 50 ml et dégazée sous azote pendant 2 heures sous faible agitation (200 tours/min). Le styrène (0,3 g) dans lequel est dissous le 2,2'-azobis(2,4-diméthylvaléronitrile) (4 mg) sont alors ajoutés dans le réacteur à l'aide d'une seringue. L'ensemble est laissé sous agitation (200 tours/min) et à la température ambiante pendant une heure. L'élévation de la température jusqu'à 70°C provoque la décomposition de l'amorceur et initie la polymérisation dans les gouttes d'émulsion. La polymérisation dure 15 heures et permet d'obtenir un latex magnétique carboxylique stable constitué par des particules de polystyrène renfermant des grains d'oxyde de fer.

### Exemple 3 : préparation de particules composites avec un copolymère amphiphile dibloc

L'émulsion préparée selon le protocole décrit dans l'Exemple 1 (i) (50 ml ; Ts = 5%) initialement stabilisée dans le Triton X405 (2 g/l) est lavée à trois reprises par séparation magnétique avec la solution de copolymère précédente (0,8 g/l, pH8). Ce copolymère a été obtenu selon un procédé de polymérisation par voie radicalaire contrôlée. Il possède une masse d'environ 20 000 g/mol. Le bloc polystyrène représente environ 10% de la masse du polymère. A chaque lavage, le surnageant limpide est éliminé et remplacé par un volume égal de solution contenant le copolymère. Après avoir été filtré sur un filtre nylon de 1 µm, l'émulsion est introduite dans un réacteur de 50 ml et dégazée sous azote pendant 2 heures sous faible agitation (200 tours/min). Le styrène (0,3 g) et le 2,2'-azobis(2,4-diméthylvaléronitrile) (6 mg) sont alors ajoutés dans le réacteur à l'aide d'une seringue. L'ensemble est laissé sous agitation (200 tours/min) et à température ambiante pendant une heure. L'élévation de la température jusqu'à 70°C provoque la décomposition de l'amorceur et initie la polymérisation dans les gouttes d'émulsion. La polymérisation dure 15 heures et permet d'obtenir un latex magnétique carboxylique stable constitué par des particules de polystyrène renfermant des grains d'oxyde de fer.

### Exemple 4: préparation de particules composites avec un copolymère amphiphile dibloc

L'émulsion préparée selon le protocole décrit dans l'Exemple 1 (i) (50 ml ; Ts = 5%) initialement stabilisée dans le Triton X405 (2 g/l) est lavée à deux reprises par séparation magnétique avec la solution de copolymère précédente (0,4 g/l ; pH8). Le bloc polyacide acrylique a été obtenu par polymérisation radicalaire contrôlée. Le polymère possède une masse de 18 600 g/mol et le bloc polyéthylène représente environ 2% de cette masse. A chaque lavage, le surnageant limpide est éliminé et remplacé par un volume égal de solution contenant le copolymère. Après avoir été filtré sur un filtre nylon de 1 µm, l'émulsion est introduite dans un réacteur de 50 ml et dégazée sous azote pendant 2 heures sous faible agitation (200 tours/min). Le styrène (0,3 g) et le 2,2'-azobis(2,4-diméthylvaléronitrile) (6 mg) sont alors ajoutés dans le réacteur à l'aide d'une seringue. L'ensemble est laissé sous agitation (200 tours/min) et à température ambiante pendant une heure. L'élévation de la température jusqu'à 70°C provoque la décomposition de l'amorceur et initie la polymérisation dans les gouttes d'émulsion. La polymérisation dure 14 heures et permet d'obtenir un latex magnétique carboxylique stable constitué par des particules de polystyrène renfermant des grains d'oxyde de fer.

### Exemple 5 : préparation de particules composites avec un copolymère amphiphile « en peigne », dont la partie hydrophobe est immobilisée à la surface de la matrice polymère

### (i) Obtention du latex

20 ml de l'émulsion de départ stable et isodisperse préparée selon le protocole de l'exemple 1(i) et constituée de deux phases non miscibles : la phase A constituée de gouttelettes de 180 nm plus ou moins 5 nm de diamètre contenant des nanoparticules d'oxydes de fer de 10 nm dans de l'octane et un mélange de tensioactifs (dodécyl sulfate de sodium (SDS) et acide oléique), dispersée dans la phase aqueuse B (concentration 1%, à 0,8 fois la concentration micellaire critique (CMC) du SDS) sont placés dans un réacteur de polymérisation. L'émulsion est dégazée pendant 7 heures à l'azote. Du 2,2'-azobis(2,4-diméthyl valéronitrile), comme amorceur de la polymérisation, solubilisé dans l'hexane (215 µl à 20 g/l) est introduit dans l'émulsion. Le mélange est soumis à homogénéisation pendant une heure et 80 mg de monomères de styrène sont ensuite introduits. Les monomères de styrène diffusent à l'intérieur de la phase A pendant deux heures. La polymérisation est amorcée par chauffage de la solution à une température de 70°C et poursuivie pendant 12 heures trente minutes, sous agitation lente. Après achèvement de la polymérisation, les particules composites obtenues présentent un diamètre de 188 nm et un indice de polydispersité de l'ordre de 1,1.

### (ii) Immobilisation du copolymère amphiphile

Des particules de latex magnétiques polystyrène obtenus en (i) (45 ml) sont lavées deux fois avec une solution de polymère Coatex (PM = 20 000 g/mol ; 0,8 g/l ; pH3,4) par des séparations magnétiques successives. Le latex ainsi lavé est introduit (44 ml ; Ts = 3,8%) dans le réacteur de polymérisation. Le milieu réactionnel est dégazé deux heures par l'azote sous agitation (200 tours/min). On introduit le styrène (0,3 g) et on laisse gonfler pendant une heure sous agitation à 200 tours/min. On élève la température à 70°C puis on introduit du persulfate de potassium (KPS) (6 mg dans 1 ml d'eau milliQ). Puis on polymérise à 70°C pendant 7 heures sous agitation à 200 tours/min.

### Exemple 6 : préparation de particules composites avec un copolymère amphiphile dibloc, dont la partie hydrophobe est immobilisée à la surface de la matrice polymère

Des particules de latex magnétique polystyrène (45 ml obtenus par polymérisation in situ d'une émulsion avec un mélange classique styrène / amorceur non hydrosoluble, conformément à l'Exemple 5 (i)) sont lavées deux fois avec une solution de polymère dibloc (PM = 10 000 g/mol ; 0,8 g/l ; pH = 3,4) par des séparations magnétiques successives. Le latex ainsi lavé est introduit (40 ml ; Ts = 3,4%) dans le réacteur de polymérisation. Le milieu réactionnel est dégazé deux heures par l'azote sous agitation (200 tours/min). On introduit le styrène (0,3 g) et on laisse gonfler pendant une heure sous agitation à 200 tours/min. On élève la température à 70°C puis on introduit du persulfate de potassium (KPS) (4 mg dans 0,2 ml d'eau milliQ). Puis on polymérise à 70°C pendant 16 heures sous agitation à 200 tours/min.

### Exemple 7 : Préparation de particules composites avec un copolymère amphiphile « en peigne »

L'émulsion magnétique préparée comme indiqué dans l'exemple 1(i) (diamètre 220nm et de concentration 4% masse/volume) est stabilisée avec un copolymère amphiphile en peigne polyacide acrylique modifié par des chaînons hydrophobes (PM = 50000 g/mol), constitué par 80% de motifs acide acrylique et 20% de motifs méthacrylate de polyoxyalkylène/aryle (Coatex M883), à une concentration de 0,05%, et placée dans un réacteur de polymérisation. Six mg d'AIBN (2,2'-azobisisobutyronitrile), 360 mg de styrène et 240 mg de divinylbenzène sont ajoutés et incubés pendant 1 heure à 20°C. La polymérisation est engendrée par élévation de la température à 70°C pendant 13 heures. Le latex magnétique final a une taille de l'ordre de 240 nm.

### Exemple 8 : Préparation de particules composites

L'émulsion magnétique telle que préparée dans l'exemple 1(i) est stabilisée dans le Triton X-405 à 0.2%, puis est lavée à trois reprises avec une solution du polymère tel que décrit dans l'exemple 7 (Coatex M883 ; PM = 50000 g/mol) à une concentration de 0.05%.

### Exemple 9 : Greffage de la streptavidine

On a ajouté 100 µl de tampon phosphate (10 mM, pH 6.8) dans 0.5 mg des particules telles qu'obtenues dans les exemples 7 et 8 précédents. On a agité puis aimanté les particules. On a repris les particules dans 50 µl de tampon phosphate. Dans un tube Eppendorf, on a mélangé 15 µl de streptavidine (5mg/ml) avec 50 µl de carbodiimide hydrosoluble (1mg/ml). On a agité 2 minutes à 300 tour/min puis on a ajouté les particules dans le tube. On a incubé à 40°C et 1000 tour/min pendant 30 minutes puis on a ajouté 100 µl de tampon de blocage (PBS+Triton X-405+ éthanolamine). On a incubé à 40°C et 1000 tour/min pendant 30 minutes. On a alors aimanté les particules puis on les a reprises dans 100 µl de tampon de stockage (PBS+Triton X-405). On a effectué deux nouveaux lavages avec le tampon de stockage. Lors d'un dernier lavage, on a repris les particules dans 50 µl de tampon de stockage.

### Exemple 10 : Greffage d'un oligonucléotide biotinylé sur le conjugué particule-streptavidine

On a aimanté les particules de 0.25 mg des conjugués tels qu'obtenus dans l'exemple 9 précédent et on les a reprises dans 50 µl de tampon TE NaCl (Tris 10mM, EDTA 1mM, NaCl 1M, Triton X-405 0.05%, ADN de saumon). On a ajouté 2,2.10¹³ copies d'oligonucléotide (ODN) biotinylé. On a incubé à 37°C et 1000 tour/min pendant 30 minutes. On a aimanté les particules et on les a reprises dans 50 µl de tampon TE NaCl.

### Exemple 11 : Hybridation et révélation du couplage

On a ajouté 100 µl de tampon TE NaCl à 40 µg des conjugués tels qu'obtenus dans l'exemple 10 précédent. On a ajouté 18.10¹⁰ copies d'oligonucléotide cible et 18.10¹⁰ copies d'oligonucléotide de détection marqué par de la peroxydase de raifort. On a incubé à 37°C et 1000 tour/min pendant 30 minutes. On a lavé à trois reprises dans le tampon TE NaCl. On a ajouté 100 µl d'o-phénylènediamine (OPD) (dilution de 5mg d'OPD dans 2,5 ml de diluant Coloreia). On a incubé pendant 3 minutes. On a ajouter 100 µl de H₂SO₄ 1N, puis on a aimanté les particules. On a prélevé 100 µl de surnageant et on a analysé son intensité à 492 nm. Le témoin négatif est composé de conjugués sans cible. Les résultats sont indiqués dans le tableau suivant :

| | témoin négatif | Test-1 | Test-2 |
|---|---|---|---|
| Conjugués de l'exemple 8 | 0,266 | 0,848 | 0,871 |
| Conjugués de l'exemple 9 | 0,242 | 0,822 | 0,824 |

## Revendications

1. Particules composites comprenant un coeur en un polymère hydrophobe et des nanoparticules inorganiques, ledit polymère hydrophobe constituant une matrice polymère à l'intérieur de laquelle les nanoparticules inorganiques sont stabilisées et distribuées de manière homogène, lesdites particules étant **caractérisées en ce qu'**elles sont au moins partiellement entourées par un copolymère amphiphile comportant une partie hydrophobe et une partie hydrophile et dont la partie hydrophobe est au moins partiellement immobilisée sur ou dans ladite matrice polymère,
- la matrice polymère étant une matrice de type polymère vinylaromatique hydrophobe choisie parmi les homopolymères de monomères vinylaromatiques insolubles dans l'eau, tels que styrène, méthylstyrène, éthylstyrène, tertio-butyl-styrène, vinyltoluène, ainsi que les copolymères de ces monomères entre eux et/ou avec d'autres comonomères, tels que les acrylates d'alkyle et les méthacrylates d'alkyle dans lesquels le groupement alkyle comprend de 3 à 10 atomes de carbone, les esters d'acides éthyléniques possédant 4 ou 5 atomes de carbone et d'alkyle possédant 1 à 8 atomes de carbone, les acides méthacryliques, les dérivés styréniques, les composés diéniques,
- la partie hydrophobe du copolymère amphiphile étant choisie parmi les polystyrènes, les polyalkyles tels que le polyéthylène, les chaînes d'acides gras,
- la partie hydrophile du copolymère amphiphile étant choisie parmi les polyacides acryliques, les polysulfates, les polyamines, les polyamides les polysaccharides.

2. Particules selon la revendication 1, **caractérisées en ce qu'**elles sont complètement entourées par un copolymère amphiphile.

3. Particules selon la revendication 1 ou 2, **caractérisées en ce que** la totalité de la partie hydrophobe du copolymère amphiphile est immobilisée sur ou dans la matrice polymère.

4. Particules selon l'une quelconque des revendications 1 à 3, **caractérisées en ce que** le copolymère amphiphile est choisi parmi les polymères en bloc, les polymères séquencés, les polymères ramifiés, les polymères en peigne.

5. Particules selon la revendication 4, **caractérisées en ce qu'**elles présentent des groupements fonctionnels réactifs à leur surface, tels que des groupements carboxylique, amine, thiol, aldéhyde, hydroxyl, tosyl, hydrazine, susceptibles de réagir avec au moins un ligand.

6. Particules selon la revendication 1, **caractérisées en ce que** les nanoparticules sont constituées de matériaux inorganiques choisis parmi les oxydes métalliques de fer, de titane, de cobalt, de zinc, de cuivre, de manganèse, de nickel ; la magnétite ; l'hématite ; les ferrites telles que les ferrites de manganèse, de nickel, de manganèse-zinc ; les alliages de cobalt, de nickel.

7. Particules selon la revendication 6, **caractérisées en ce que** les matériaux inorganiques sont choisis parmi les oxydes métalliques magnétisables, tels que l'oxyde de fer, la magnétite et l'hématite.

8. Particules selon la revendication 6 ou 7, **caractérisées en ce que** les matériaux inorganiques représentent de 5 à 95%, de préférence de 10 à 90%, de manière encore plus préférée de 20 à 90%, et avantageusement de 25 à 85% en masse par rapport à la masse totale desdites particules.

9. Particules selon la revendication 1, **caractérisées en ce que** les nanoparticules inorganiques sont stabilisées par des agents de stabilisation choisis parmi les groupements ioniques, les chaînes polymère, les agents tensioactifs, les agents tensioactifs fonctionnels, tels que les acides gras ou les dérivés d'acides gras, et les mélanges d'agents tensioactifs.

10. Particules selon la revendication 9, **caractérisées en ce que** les agents tensioactifs sont choisis parmi le dodécyl sulfate de sodium et les mélanges de dodécyl sulfate de sodium et d'acide oléique.

11. Particules selon l'une quelconque des revendications 1 à 10, **caractérisées en ce que** le polymère est un polymère réticulé.

12. Particules selon les revendications précédentes, **caractérisées en ce qu'**elles comprennent de plus un marqueur radioactif.

13. Particules selon l'une quelconque des revendications 1 à 12, **caractérisées en ce qu'**elles présentent un diamètre de l'ordre de 50 à 1000 nm, de préférence d'environ 100 à 500 nm et avantageusement d'environ 100 à 250 nm.

14. Procédé de préparation de particules composites selon l'une quelconque des revendications 1 à 13 et pour laquelle la partie hydrophobe du copolymère amphiphile est au moins partiellement immobilisée dans la matrice polymère, **caractérisé en ce que**
(i) on dispose d'une émulsion de départ stable et isodisperse constituée de deux phases non miscibles, une phase A hydrophobe constituée de gouttelettes contenant des nanoparticules inorganiques dispersées dans une phase organique contenant un agent tensioactif ; et une phase B hydrophile dans laquelle la phase A est dispersée ;
(ii) on introduit dans la phase B des monomères hydrophobes et au moins un agent amorceur; et
(iii) on polymérise lesdits monomères hydrophobes à l'intérieur de la phase A,
ledit procédé étant **caractérisé en ce que**, avant l'étape (iii), on introduit en outre, dans la phase B, au moins un copolymère amphiphile,
la phase A étant une phase comprenant un hydrocarbure aliphatique ou cyclique choisi parmi les composés comprenant de 5 à 12 atomes de carbone, leurs isomères et leurs mélanges ou une phase comprenant tout ou partie d'un composé organique polymérisable par voie radicalaire choisi parmi les monomères vinylaromatiques insolubles dans l'eau, tels que styrène, méthylstyrène, éthylstyrène, tertio-butyl-styrène, vinyltoluène, ainsi que les copolymères de ces monomères entre eux,
la phase B étant une phase aqueuse, telle que de l'eau,
les monomères hydrophobes étant choisis parmi les monomères vinylaromatiques tels que styrène, méthylstyrène, éthylstyrène, tertio-butyl-styrène, amino-méthylstyrène, vinyltoluène, seuls ou en mélange, ou en mélange avec d'autres monomères polymérisables insolubles dans l'eau tels que les acrylates, les méthacrylates d'alkyle, les esters d'acides éthyléniques et d'alkyle, les acides méthacryliques, les dérivés styréniques, les acides éthyléniques, les composés diéniques.

15. Procédé selon la revendication 14, **caractérisé en ce qu'**on introduit le copolymère amphiphile avant ou après l'étape (ii).

16. Procédé de préparation de particules composites selon l'une quelconque des revendications 1 à 13 et pour laquelle la partie hydrophobe du copolymère amphiphile est au moins partiellement immobilisée sur la matrice polymère, **caractérisé en ce que**
(i) on dispose d'une émulsion de départ stable et isodisperse constituée de deux phases non miscibles, une phase A hydrophobe constituée de gouttelettes contenant des nanoparticules inorganiques dispersées dans une phase organique contenant un agent tensioactif ; et une phase B hydrophile dans laquelle la phase A est dispersée ;
(ii) on introduit dans la phase B des monomères hydrophobes et au moins un agent amorceur ; et
(iii) on polymérise une fraction desdits monomères hydrophobes à l'intérieur de la phase A,
ledit procédé étant **caractérisé en ce que**, avant l'étape (iii) ou après l'étape (iii), on introduit en outre, dans la phase B, au moins un copolymère amphiphile, puis, selon une étape (iv), on polymérise la fraction restante des monomères hydrophobes à la surface de la matrice polymère,
la phase A étant une phase comprenant un hydrocarbure aliphatique ou cyclique choisi parmi les composés comprenant de 5 à 12 atomes de carbone, leurs isomères et leurs mélanges ou une phase comprenant tout ou partie d'un composé organique polymérisable par voie radicalaire choisi parmi les monomères vinylaromatiques insolubles dans l'eau, tels que styrène, méthylstyrène, éthylstyrène, tertio-butyl-styrène, vinyltoluène, ainsi que les copolymères de ces monomères entre eux,
la phase B étant une phase aqueuse, telle que de l'eau,
les monomères hydrophobes étant choisis parmi les monomères vinylaromatiques tels que styrène, méthylstyrène, éthylstyrène, tertio-butyl-styrène, amino-méthylstyrène, vinyltoluène, seuls ou en mélange, ou en mélange avec d'autres monomères polymérisables insolubles dans l'eau tels que les acrylates, les méthacrylates d'alkyle, les esters d'acides éthyléniques et d'alkyle, les acides méthacryliques, les dérivés styréniques, les acides éthyléniques, les composés diéniques.

17. Procédé selon la revendication 16, **caractérisé en ce que** lorsqu'on introduit le copolymère amphiphile avant l'étape (iii), on l'introduit avant ou après l'étape (ii):

18. Procédé selon la revendication 14 ou 16, **caractérisé en ce que** le copolymère amphiphile est choisi parmi les polymères en bloc, les polymères séquencés, les polymères ramifiés, les polymères en peigne.

19. Procédé selon la revendication 14 ou 16, **caractérisé en ce que** on ajoute au monomère ou au mélange de monomères un agent de réticulation hydrophobe, tel qu'un monomère réticulant de type divinylbenzène, diméthacrylate, en particulier diméthacrylate de vinyle.

20. Procédé selon la revendication 14 ou 16, **caractérisé en ce que** l'agent amorceur est choisi parmi les amorceurs organosolubles ou faiblement solubles dans la phase hydrophile, et les amorceurs solubles dans la phase hydrophile.

21. Procédé selon la revendication 20, **caractérisé en ce que** l'agent amorceur organosoluble est choisi parmi amorceurs de type azobis, tels que les 2,2'-azobis(2,4-diméthylvaléronitrile), 2,2'-azobis(4-méthoxy-2,4-diméthylvaléronitrile), 2,2'-azobis(2-cyclopropylpropionitrile), 2,2'-azobis(2-méthylpropionitrile), 2,2'-azobis(2-méthytbutyronitrite), 1,1'-azobis (cyclohexane-1-carbonitrile), 1-((1-cyano-1-méthyléthyl)azo)formamide, 2-phénylazo-4-méthoxy-2,4-diméthyl-valéronitrile, diméthyl-2,2'-azobis(2-méthylpropionate, 4,4'-azobis(4-acide cyanovalérique) et 2,2'-azobis(2-(hydroxyméthyl)propionitrile) et l'amorceur faiblement soluble ou soluble dans la phase hydrophile est choisi parmi les amorceurs radicalaires de type peroxyde, tels que les peroxydes, les hydroperoxydes, en particulier l'hydroperoxyde de cumène, et les amorceurs de type persel, tels que les persulfates, en particulier le perulfate d'ammonium, le persulfate de sodium et le persulfate de potassium.

22. Procédé selon les revendications 14 ou 16 et 20 ou 21, **caractérisé en ce que** l'agent amorceur est introduit dans la phase hydrophile soit simultanément à l'introduction des monomères hydrophobes, soit avant, soit après l'introduction desdits monomères.

23. Procédé selon la revendication 14 ou 16, **caractérisé en ce que** la polymérisation (iii) et/ou (iv) est effectuée par chauffage jusqu'à une température de 60 à 90°C, de préférence de 70°C.

24. Procédé selon la revendication 14 ou 16, **caractérisé en ce que** la polymérisation (iii) est effectuée par photochimie.

25. Procédé selon la revendication 14 ou 16, **caractérisé en ce que** l'hydrocarbure est choisi parmi le pentane, l'hexane, l'heptane, l'octane, le nonane, le décane, le undécane, le dodécane, et le composé organique polymérisable par voie radicalaire est choisi parmi les parmi le styrène, méthylstyrène, éthylstyrène, tertio-butyl-styrène, vinyltoluène, ainsi que les copolymères de ces monomères entre eux.

26. Procédé selon la revendication 14 ou 16, **caractérisé en ce qu'**il comprend de plus une étape de fonctionalisation des particules composites par pénétration dans la phase A de groupements réactifs fonctionnels apportés par des monomères faiblement hydrophiles susceptibles de polymériser avec les monomères hydrophobes.

27. Procédé selon la revendication 26, **caractérisé en ce que** les groupements réactifs fonctionnels sont des groupements acides apportés par des monomères faiblement hydrophiles choisis parmi les monomères des acides acryliques, méthacryliques, éthyléniques, sulfoniques et les monomères polymérisables choisis parmi les monomères vinylaromatiques insolubles dans l'eau, tels que styrène, méthylstyrène, éthylstyrène,tertio-butyl-styrène, vinyltoluène, ainsi que les copolymères de ces monomères entre eux, seuls ou en mélange avec d'autres monomères hydrophobes.

28. Procédé selon les revendications 14 ou 16 et 26 ou 27, **caractérisé en ce que** lesdits monomères faiblement hydrophiles sont apportés dans la phase A, soit simultanément à l'introduction des monomères hydrophobes, soit avant, soit après l'introduction desdits monomères.

29. Procédé selon la revendication 14 ou 16, **caractérisé en ce qu'**il comprend de plus après l'étape de polymérisation (iii), une étape d'évaporation totale ou partielle de la phase A.

30. Conjugués **caractérisés en ce qu'**ils sont constitués par des particules composites telles que définies à la revendication 5, dont les groupements fonctionnels ont interagi, directement ou indirectement, avec au moins un ligand choisi parmi un anticorps, un fragment d'anticorps, une protéine, un polypeptide, une enzyme, un polynucléotide, un fragment d'acide nucléique et la biotine.

31. Réactif comprenant en outre au moins un conjugué tel que défini dans la revendication 30.

32. Conjugués **caractérisés en ce qu'**ils sont constitués par des particules composites telles que définies à la revendication 5, dont les groupements fonctionnels ont interagi, directement ou indirectement, avec au moins un ligand choisi parmi les molécules cages, les agents chélatants et les catalyseurs.

33. Conjugués **caractérisés en ce qu'**ils sont constitués par des particules composites telles que définies à la revendication 5, dont les groupements fonctionnels ont interagi, directement ou indirectement, avec au moins un ligand choisi parmi les substances médicamenteuses, les sondes anti-sens, les agents réparateurs de gènes, les agents bloquant ou inhibant une activité protéique.

34. Utilisation des conjugués tels que définis dans la revendication 33 dans une composition thérapeutique.

## Patentansprüche

1. Verbundstoffpartikel, umfassend einen Kern aus einem hydrophoben Polymer und anorganische Nanopartikel, wobei das hydrophobe Polymer eine Polymermatrix darstellt, in deren Inneren die anorganischen Nanopartikel stabilisiert und auf homogene Weise verteilt sind, wobei die Partikel **dadurch gekennzeichnet** sind, dass sie mindestens teilweise von einem amphiphilen Copolymer umgeben sind, das einen hydrophoben Teil und einen hydrophilen Teil umfasst, und wobei der hydrophobe Teil mindestens teilweise auf oder in der Polymermatrix immobilisiert ist,
- wobei die Polymermatrix eine Matrix vom Typ hydrophobes vinylaromatisches Polymer ist, ausgewählt aus den Homopolymeren von wasserunlöslichen vinylaromatischen Monomeren wie z.B. Styrol, Methylstyrol, Ethylstyrol, tertiäres Butylstyrol, Vinyltoluen, ebenso wie die Copolymere dieser Monomere untereinander und/oder mit anderen Comonomeren, wie z.B. die Alkylacrylate und die Alkylmethacrylate, in denen die Alkylgruppe von 3 bis 10 Kohlenstoffatome umfasst, wobei die Ethylensäureester 4 oder 5 Kohlenstoffatome aufweisen und die Alkylester 1 bis 8 Kohlenstoffatome aufweisen, die Methacrylsäuren, die Styrolderivate, die Dienverbindungen,
- wobei der hydrophobe Teil des amphiphilen Copolymers ausgewählt ist aus den Polystyrolen, den Polyalkylen wie z.B. dem Polyethylen, den Fettsäureketten,
- wobei der hydrophile Teil des amphiphilen Copolymers ausgewählt ist aus den Polyacrylsäuren, den Polysulfaten, den Polyaminen, den Polyamiden, den Polysacchariden.

2. Partikel nach Anspruch 1, **dadurch gekennzeichnet, dass** sie vollständig von einem amphiphilen Copolymer umgeben sind.

3. Partikel nach Anspruch 1 oder 2, **dadurch gekennzeichnet, dass** die Gesamtheit des hydrophoben Teils des amphiphilen Copolymers auf oder in der Polymermatrix immobilisiert ist.

4. Partikel nach einem der Ansprüche 1 bis 3, **dadurch gekennzeichnet, dass** das amphiphile Copolymer ausgewählt ist aus den Blockpolymeren, den sequenzierten Polymeren, den verzweigen Polymeren, den Kammpolymeren.

5. Partikel nach Anspruch 4, **dadurch gekennzeichnet, dass** sie funktionelle Gruppen aufweisen, die auf ihrer Oberfläche reaktionsfähig sind, wie z.B. Carboxyl-, Amin-, Thiol-, Aldehyd-, Hydroxyl-, Tosyl-, Hydrazingruppen, die mit mindestens einem Liganden reagieren können.

6. Partikel nach Anspruch 1, **dadurch gekennzeichnet, dass** die Nanopartikel aus anorganischen Materialien bestehen, ausgewählt aus den metallischen Eisen-, Titan-, Kobalt-, Zink-, Kupfer-, Mangan-, Nickeloxyden; Magnetit; Hämatit; Ferriten wie z.B. den Mangan-, Nickel-, Mangan-Zink-Ferriten; den Kobalt-, Nickellegierungen.

7. Partikel nach Anspruch 6, **dadurch gekennzeichnet, dass** die anorganischen Materialien ausgewählt sind aus den magnetisierbaren metallischen Oxiden wie z.B. dem Einsenoxid, dem Magnetit und dem Hämatit.

8. Partikel nach Anspruch 6 oder 7, **dadurch gekennzeichnet, dass** die anorganischen Materialien von 5 bis 95, vorzugsweise von 10 bis 90, noch bevorzugter von 20 bis 90, und am vorteilhaftesten von 25 bis 85 Massenprozent mit Bezug auf die gesamte Masse der Partikel darstellen.

9. Partikel nach Anspruch 1, **dadurch gekennzeichnet, dass** die anorganischen Nanopartikel durch Stabilisierungsmittel stabilisiert sind, ausgewählt aus den ionischen Gruppen, den Polymerketten, den Tensiden, den funktionellen Tensiden wie z.B. den Fettsäuren oder den Fettsäurederivaten und den Mischungen von Tensiden.

10. Partikel nach Anspruch 9, **dadurch gekennzeichnet, dass** die Tenside ausgewählt sind aus dem Natriumdodecylsulfat und den Mischungen aus Natriumdodecylsulfat und Oleinsäure.

11. Partikel nach einem der Ansprüche 1 bis 10, **dadurch gekennzeichnet, dass** das Polymer ein vernetztes Polymer ist.

12. Partikel nach den vorhergehenden Ansprüchen, **dadurch gekennzeichnet, dass** sie außerdem einen radioaktiven Marker umfassen.

13. Partikel nach einem der Ansprüche 1 bis 12, **dadurch gekennzeichnet, dass** sie einen Durchmesser im Bereich von 50 bis 1000 nm, vorzugsweise von ungefähr 100 bis 500 nm und vorteilhafterweise von ungefähr 100 bis 250 nm aufweisen.

14. Verfahren zur Herstellung von Verbundstoffpartikeln nach einem der Ansprüche 1 bis 13, und für das der hydrophobe Teil des amphiphilen Copolymers mindestens teilweise in der Polymermatrix immobilisiert ist, **dadurch gekennzeichnet, dass**
(i) eine stabile und isosidperse Ausgangsemulsion vorhanden ist, die aus zwei nicht mischbaren Phasen besteht, einer hydrophoben Phase A, die aus Tröpfchen mit anorganischen Nanopartikeln besteht, die in einer organischen Phase mit einem Tensid dispergiert sind; und einer hydrophilen Phase B, in der die Phase A dispergiert ist;
(ii) in die Phase B hydrophobe Monomere und mindestens ein Aktivator eingeführt werden; und
(iii) die hydrophoben Monomere im Inneren der Phase A polymerisiert werden,
wobei das Verfahren **dadurch gekennzeichnet** ist, dass vor dem Schritt (iii) außerdem in die Phase B mindestens ein amphiphiles Copolymer eingeführt wird,
wobei die Phase A eine Phase ist, die einen aliphatischen oder cyclischen Kohlenwasserstoff umfasst, ausgewählt aus den Verbindungen, die 5 bis 12 Kohlenstoffatome umfassen, wobei ihre Isomere und ihre Mischungen oder eine Phase die Gesamtheit oder einen Teil einer organischen Verbindung umfassen, die auf radikalischem Weg polymerisierbar sind, ausgewählt aus den wasserunlöslichen vinylaromatischen Monomeren wie z.B. Styrol, Methylstyrol, Ethylstyren, tertiäres Butylstyrol, Vinyltoluen, ebenso wie die Copolymere dieser Monomere untereinander,
wobei die Phase B eine wässrige Phase wie z.B. Wasser ist,
wobei die hydrophoben Monomere ausgewählt sind aus den vinylaromatischen Monomeren wie z.B. Styrol, Methylstyrol, Ethylstyrol, tertiäres Butylstyrol, Aminomethylstyrol, Vinyltoluen, alleine oder in Mischung, oder in Mischung mit anderen wasserunlöslichen polymerisierbaren Monomeren wie z.B. den Acrylaten, den Alkylmethacrylaten, den Ethylen- und Alkylsäureestern, den Methacrylsäuren, den Styrolderivaten, den Ethylensäuren, den Dienverbindungen.

15. Verfahren nach Anspruch 14, **dadurch gekennzeichnet, dass** das amphiphile Copolymer vor oder nach Schritt (ii) eingeführt wird.

16. Verfahren zur Herstellung von Verbundstoffpartikeln nach einem der Ansprüche 1 bis 13, und für das der hydrophobe Teil des amphiphilen Copolymers mindestens teilweise auf der Polymermatrix immobilisiert ist, **dadurch gekennzeichnet, dass**:
(i) eine stabile und isosidperse Ausgangsemulsion aus zwei nicht mischbaren Phasen vorhanden ist, einer hydrophoben Phase A, die aus Tröpfchen mit anorganischen Nanopartikeln besteht, die in einer organischen Phase mit einem Tensid dispergiert sind; und einer hydrophilen Phase B, in der die Phase A dispergiert ist;
(ii) in die Phase B hydrophobe Monomere und mindestens ein Aktivator eingeführt werden; und
(iii) eine Fraktion der hydrophoben Monomere im Inneren der Phase A polymerisiert wird,
wobei das Verfahren **dadurch gekennzeichnet** ist, dass vor dem Schritt (iii) oder nach dem Schritt (iii) außerdem in die Phase B mindestens ein amphiphiles Copolymer eingeführt wird, dann, nach Schritt (iv), die restliche Fraktion der hydrophoben Monomere auf der Oberfläche der Polymermatrix polymerisiert wird.
wobei die Phase A eine Phase ist, die einen aliphatischen oder cyclischen Kohlenwasserstoff umfasst, ausgewählt aus den Verbindungen, die 5 bis 12 Kohlenstoffatome umfassen, wobei ihre Isomere und ihre Mischungen oder eine Phase die Gesamtheit oder einen Teil einer organischen Verbindung umfassen, die auf radikalischem Weg polymerisierbar ist, ausgewählt aus den wasserunlöslichen vinylaromatischen Monomeren wie z.B. Styrol, Methylstyrol, Ethylstyrol, tertiäres Butylstyrol, Vinyltoluen, ebenso wie die Copolymere dieser Monomere untereinander,
wobei die Phase B eine wässrige Phase wie z.B. Wasser ist,
wobei die hydrophoben Monomere ausgewählt sind aus den vinylaromatischen Monomeren wie z.B. Styrol, Methylstyrol, Ethylstyren, tertiäres Butylstyrol, Aminomethylstyrol, Vinyltoluen, alleine oder in Mischung, oder in Mischung mit anderen wasserunlöslichen polymerisierbaren Monomeren wie z.B. den Acrylaten, den Alkylmethacrylaten, den Ethylen- und Alkylsäureestern, den Methacrylsäuren, den Styrolderivaten, den Ethylensäuren, den Dienverbindungen.

17. Verfahren nach Anspruch 16, **dadurch gekennzeichnet, dass** wenn das amphiphile Copolymer vor dem Schritt (iii) eingeführt wird, es vor oder nach dem Schritt (ii) eingeführt wird.

18. Verfahren nach Anspruch 14 oder 16, **dadurch gekennzeichnet, dass** das amphiphile Copolymer ausgewählt ist aus den Blockpolymeren, den sequenzierten Polymeren, den verzweigen Polymeren, den Kammpolymeren.

19. Verfahren nach Anspruch 14 oder 16, **dadurch gekennzeichnet, dass** dem Monomer oder der Mischung aus Monomeren ein hydrophobes Vernetzungsmittel wie z.B. ein Vernetzungsmonomer vom Typ Divinylbenzen, Dimethacrylat, insbesondere Vinyldimethacrylat zugegeben wird.

20. Verfahren nach Anspruch 14 oder 16, **dadurch gekennzeichnet, dass** der Aktivator ausgewählt ist aus in der hydrophilen Phase organisch löslichen oder schwach löslichen Aktivatoren und in der hydrophilen Phase löslichen Aktivatoren.

21. Verfahren nach Anspruch 20, **dadurch gekennzeichnet, dass** der organisch lösliche Aktivator ausgewählt ist aus Aktivatoren vom Typ Azobis wie z.B. 2,2'-Azobis(2,4-dimethylvaleronitril), 2,2'-Azobis(4-methoxy-2,4-dimethylvaleronitril), 2,2'-Azobis(2-cyclopropylpropionitril), 2,2'-Azobis(2-methylpropionitril), 2,2'-Azobis(2-methylbutyronitril), 1,1'-Azobis(cyclohexan-1-carbonitril), 1-((1-Cyano-1-methylethyl)azo)formamid, 2-Phenylazo-4-methoxy-2,4-dimethyl-valeronitril, Dimethyl-2,2'-azobis(2-methylpropionat), 4,4'-Azobis(4-cyanovaleriansäure) und 2,2'-Azobis(2-(hydroxymethyl)propionitril), und der in der hydrophilen Phase schwach lösliche oder lösliche Aktivator ausgewählt ist aus den radikalischen Aktivatoren vom Typ Peroxid wie den Peroxiden, den Hydroperoxiden, insbesondere dem Cumolhydroperoxid und den Aktivatoren vom Typ Persalz wie z.B. den Persulfaten, insbesondere dem Ammoniumpersulfat, dem Natriumpersulfat und dem Kaliumpersulfat.

22. Verfahren nach Anspruch 14 oder 16 und 20 oder 21, **dadurch gekennzeichnet, dass** der Aktivator in die hydrophile Phase entweder gleichzeitig mit der Einführung der hydrophoben Monomere oder vor oder nach der Einführung der Monomere eingeführt wird.

23. Verfahren nach Anspruch 14 oder 16, **dadurch gekennzeichnet, dass** die Polymerisierung (iii) und/oder (iv) durch Erhitzung bis zu einer Temperatur von 60 bis 90 °C, vorzugsweise 70 °C, erfolgt.

24. Verfahren nach Anspruch 14 oder 16, **dadurch gekennzeichnet, dass** die Polymerisierung (iii) durch Fotochemie erfolgt.

25. Verfahren nach Anspruch 14 oder 16, **dadurch gekennzeichnet, dass** der Kohlenwasserstoff ausgewählt ist aus Pentan, Hexan, Heptan, Octan, Nonan, Decan, Undecan, Dodecan, und die auf radikalischem Weg polymerisierbare organische Verbindung ausgewählt ist aus Styrol, Methylstyrol, Ethylstyrol, tertiärem Butylstyrol, Vinyltoluen, ebenso wie den Copolymeren dieser Monomere untereinander,

26. Verfahren nach Anspruch 14 oder 16, **dadurch gekennzeichnet, dass** es außerdem einen Schritt des Funktionalisierens der Verbundstoffpartikel durch Eindringen in die Phase A von funktionellen Reaktionsgruppen umfasst, die durch schwach hydrophile Monomere eingebracht werden, die mit den hydrophoben Monomeren polymerisieren können.

27. Verfahren nach Anspruch 26, **dadurch gekennzeichnet, dass** die funktionellen Reaktionsgruppen Säuregruppen sind, die durch schwach hydrophile Monomere eingebracht werden, ausgewählt aus den Monomeren der Acryl-, Methacryl-, Ethylen-, Sulfonsäuren und den polymerisierbaren Monomeren, ausgewählt aus den wasserunlöslichen vinylaromatischen Monomeren wie z.B. Styrol, Methylstyrol, Ethylstyrol, tertiärem Butylstyrol, Vinyltoluen, ebenso wie den Copolymeren dieser Monomere untereinander, alleine oder in Mischung, mit anderen hydrophoben Monomeren.

28. Verfahren nach Anspruch 14 oder 16 und 26 oder 27, **dadurch gekennzeichnet, dass** die schwach hydrophilen Monomere in der Phase A entweder gleichzeitig mit der Einführung der hydrophoben Monomere oder vor oder nach der Einführung der Monomere eingeführt werden.

29. Verfahren nach Anspruch 14 oder 16, **dadurch gekennzeichnet, dass** es außerdem nach dem Schritt des Polymerisierens (iii) einen Schritt des vollständigen oder teilweisen Verdampfens der Phase A umfasst.

30. Konjugate, **dadurch gekennzeichnet, dass** sie aus Verbundstoffpartikeln, wie in Anspruch 5 definiert, bestehen, deren funktionelle Gruppen direkt oder indirekt mit mindestens einem Liganden interagiert haben, ausgewählt aus einem Antikörper, einem Fragment eines Antikörpers, einem Protein, einem Polypeptid, einem Enzym, einem Polynukleotid, einem Fragment einer Nukleinsäure und Biotin.

31. Reagens, umfassend außerdem mindestens ein Konjugat wie in Anspruch 30 definiert.

32. Konjugate, **dadurch gekennzeichnet, dass** sie aus Verbundstoffpartikeln, wie in Anspruch 5 beschrieben, bestehen, deren funktionelle Gruppen direkt oder indirekt mit mindestens einem Liganden interagiert haben, ausgewählt aus den Käfigmolekülen, den Chelatbildnern und den Katalysatoren,

33. Konjugate, **dadurch gekennzeichnet, dass** sie aus Verbundstoffpartikeln bestehen, wie in Anspruch 5 definiert, deren funktionelle Gruppen direkt oder indirekt mit mindestens einem Liganden interagiert haben, ausgewählt aus den Arzneimitteln, den Antisens-Proben, den Genreparatur-Mitteln, den Mittel, die eine Proteinaktivität blockieren oder inhibieren.

34. Verwendung der Konjugate, wie in Anspruch 33 definiert, in einer therapeutischen Zusammensetzung.

## Claims

1. Composite particles comprising a core made of a hydrophobic polymer and inorganic nanoparticles, said hydrophobic polymer constituting a polymer matrix within which the inorganic nanoparticles are stabilized and distributed homogeneously, said particles being **characterized in that** they are at least partially surrounded by an amphiphilic copolymer comprising a hydrophobic part and a hydrophilic part, the hydrophobic part of which is at least partially immobilized on or in said polymer matrix,
- the polymer matrix being a matrix of hydrophobic vinylaromatic polymer type chosen from homopolymers of water-insoluble vinylaromatic monomers, such as styrene, methylstyrene, ethylstyrene, tert-butylstyrene or vinyltoluene, and the copolymers of these monomers with one another and/or with other comonomers, such as alkyl acrylates and methacrylates in which the alkyl group comprises from 3 to 10 carbon atoms, alkyl esters of ethylenic acids, the ethylenic acid possessing 4 or 5 carbon atoms and the alkyl group possessing 1 to 8 carbon atoms, methacrylic acids, styrene derivatives or diene compounds,
- the hydrophobic part of the amphiphilic copolymer being chosen from polystyrenes, polyalkyls, such as polyethylene, or fatty acid chains,
- the hydrophilic part of the amphiphilic copolymer being chosen from poly(acrylic acid)s, polysulfates, polyamines, polyamides or polysaccharides.

2. The particles as claimed in claim 1, **characterized in that** they are completely surrounded by an amphiphilic copolymer.

3. The particles as claimed in claim 1 or 2, **characterized in that** all the hydrophobic part of the amphiphilic copolymer is immobilized on or in the polymer matrix.

4. The particles as claimed in any one of claims 1 to 3, **characterized in that** the amphiphilic copolymer is chosen from block polymers, branched polymers or comb polymers.

5. The particles as claimed in claim 4, **characterized in that** they have reactive functional groups at their surface, such as carboxyl, amine, thiol, aldehyde, hydroxyl, tosyl or hydrazine groups, capable of reacting with at least one ligand.

6. The particles as claimed in claim 1, **characterized in that** the nanoparticles are composed of inorganic materials chosen from iron, titanium, cobalt, zinc, copper, manganese or nickel metal oxides; magnetite; hematite; ferrites, such as manganese, nickel or manganese-zinc ferrites; or alloys of cobalt, of nickel.

7. The particles as claimed in claim 6, **characterized in that** the inorganic materials are chosen from magnetizable metal oxides, such as iron oxide, magnetite and hematite.

8. The particle as claimed in claim 6 or 7, **characterized in that** the inorganic materials represent from 5 to 95%, preferably from 10 to 90%, most preferably from 20 to 90%, and advantageously from 25 to 85%, by mass, with respect to the total mass of said particle.

9. The particles as claimed in claim 1, **characterized in that** the inorganic nanoparticles are stabilized by stabilizing agents chosen from ionic groups, polymeric chains, surface-active agents, functional surface-active agents, such as fatty acids or fatty acid derivatives, and mixtures of surface-active agents.

10. The particles as claimed in claim 9, **characterized in that** the surface-active agents are chosen from sodium dodecyl sulfate and mixtures of sodium dodecyl sulfate and of oleic acid.

11. The particles as claimed in any one of claims 1 to 10, **characterized in that** the polymer is a crosslinked polymer.

12. The particles as claimed in the preceding claims, **characterized in that** the composite particle comprises a radioactive tracer.

13. The particles as claimed in any one of claims 1 to 12, **characterized in that** the composite particle has a diameter of the order of 50 to 1000 nm, preferably of the order of about 100 to 500 nm, and advantageously of about 100 to 250 nm.

14. A process for the preparation of the composite particles as claimed in any one of claims 1 to 13 for which the hydrophobic part of the amphiphilic copolymer is at least partially immobilized in the polymer matrix, **characterized in that** (i) a stable and isodisperse starting emulsion composed of two immiscible phases, a hydrophobic phase A composed of droplets comprising inorganic nanoparticles dispersed in an organic phase comprising a surface-active agent and a hydrophilic phase B in which the phase A is dispersed, is present;
(ii) hydrophobic monomers and at least one initiator are introduced into the phase B; and
(iii) said hydrophobic monomers are polymerized inside the phase A, said process being **characterized in that**, before stage (iii), at least one amphiphilic copolymer is additionally introduced into the phase B,
the phase A being a phase comprising an aliphatic or cyclic hydrocarbon chosen from compounds comprising from 5 to 12 carbon atoms, their isomers and their mixtures, or a phase comprising all or part of an organic compound which can be polymerized by the radical route chosen from water-insoluble vinylaromatic monomers, such as styrene, methylstyrene, ethylstyrene, tert-butylstyrene or vinyltoluene, and the copolymers of these monomers with one another,
the phase B being an aqueous phase, such as water,
the hydrophobic monomers being chosen from vinylaromatic monomers, such as styrene, methylstyrene, ethylstyrene, tert-butylstyrene, aminomethylstyrene or vinyltoluene, alone or as a mixture, or alternatively as a mixture with other water-insoluble polymerizable monomers, such as alkyl acrylates, alkyl methacrylates, alkyl esters of ethylenic acids, methacrylic acids, styrene derivatives, ethylenic acids or diene compounds.

15. The process as claimed in claim 14, **characterized in that** the amphiphilic copolymer is introduced before or after stage (ii).

16. A process for the preparation of the composite particle as claimed in any one of claims 1 to 13 and for which the hydrophobic part of the amphiphilic copolymer is at least partially immobilized on the polymer matrix, **characterized in that** (i) a stable and isodisperse starting emulsion composed of two immiscible phases, a hydrophobic phase A composed of droplets comprising inorganic nanoparticles dispersed in an organic phase comprising a surface-active agent and a hydrophilic phase B in which the phase A is dispersed, is present;
(ii) hydrophobic monomers and at least one initiator are introduced into the phase B; and
iii) a fraction of said hydrophobic monomers is polymerized inside the phase A, said process being **characterized in that**, before stage (iii) or after stage (iii), at least one amphiphilic copolymer is additionally introduced into the phase B and then, according to a stage (iv), the remaining fraction of the hydrophobic monomers is polymerized at the surface of the polymer matrix,
the phase A being a phase comprising an aliphatic or cyclic hydrocarbon chosen from compounds comprising from 5 to 12 carbon atoms, their isomers and their mixtures, or a phase comprising all or part of an organic compound which can be polymerized by the radical route chosen from water-insoluble vinylaromatic monomers, such as styrene, methylstyrene, ethylstyrene, tert-butylstyrene or vinyltoluene, and the copolymers of these monomers with one another,
the phase B being an aqueous phase, such as water,
the hydrophobic monomers being chosen from vinylaromatic monomers, such as styrene, methylstyrene, ethylstyrene, tert-butylstyrene, aminomethylstyrene or vinyltoluene, alone or as a mixture, or alternatively as a mixture with other water-insoluble polymerizable monomers, such as alkyl acrylates, alkyl methacrylates, alkyl esters of ethylenic acids, methacrylic acids, styrene derivatives, ethylenic acids or diene compounds.

17. The process as claimed in claim 16, **characterized in that**, when the amphiphilic copolymer is introduced before stage (iii), it is introduced before or after stage (ii).

18. The process as claimed in claim 14 or 16, **characterized in that** the amphiphilic copolymer is chosen from block polymers, branched polymers or comb polymers.

19. The process as claimed in claim 14 or 16, **characterized in that** a hydrophobic crosslinking agent, such as a crosslinking monomer of divinylbenzene or dimethacrylate type, is added to the monomer or to the mixture of monomers.

20. The process as claimed in claim 14 or 16, **characterized in that** the initiator is chosen from organosoluble initiators, initiators which are weakly soluble in the hydrophilic phase and initiators which are soluble in the hydrophilic phase.

21. The process as claimed in claim 20, **characterized in that** the organosoluble initiator is chosen from initiators of azobis type, such as 2,2'-azobis(2,4-dimethylvaleronitrile), 2,2'-azobis(4-methoxy-2,4-dimethylvaleronitrile), 2,2'-azobis(2-cyclopropylpropionitrile), 2,2'-azobis(2-methyl-propionitrile), 2,2'-azobis(2-methylbutyronitrile), 1,1'-azobis(cyclohexane-1-carbonitrile), 1-((I-cyano-1-methylethyl)azo)formamide,2-phenylazo-4-methoxy-2,4-dimethylvaleronitrile , dimethyl 2,2'-azobis(2-methylpropionate), 4,4'-azobis(4-cyanovaleric acid) and 2,2'-azobis(2-(hydroxymethyl)propionitrile), and the initiator which is weakly soluble or soluble in the hydrophilic phase is chosen from radical initiators of peroxide type, such as peroxides or hydroperoxides, in particular cumene hydroperoxide, and initiators of persalt type, such as persulfates, in particular ammonium persulfate, sodium persulfate and potassium persulfate.

22. The process as claimed in claims 14, 16, 20, or 21, **characterized in that** the initiator is introduced into the hydrophilic phase either simultaneously with the introduction of the hydrophobic monomers or before or alter the introduction of said monomers.

23. The process as claimed in claim 14 or 16, **characterized in that** the polymerization (iii) and/or (iv) is carried out by heating up to a temperature of 60 to 90° C, preferably up to 70°C.

24. The process as claimed in claim 14 or 16, **characterized in that** the polymerization (iii) is carried out by photochemistry.

25. The process as claimed in claims 14 or 16, **characterized in that** the hydrocarbon is chosen from pentane, hexane, heptane, octane, nonane, decane, undecane or dodecane and the organic compound which can be polymerized by the radical route is chosen from the from styrene, methylstyrene, ethylstyrene, tert-butylstyrene or vinyltoluene, and the copolymers of these monomers with one another.

26. The process as claimed in claim 14 or 16, **characterized in that** it additionally comprises a stage of functionalization of the composite particle by penetration into the phase A of reactive functional groups introduced by weakly hydrophilic monomers capable of polymerizing with the hydrophobic monomers.

27. The process as claimed in claim 26, **characterized in that** the reactive functional groups are acid groups introduced by weakly hydrophilic monomers chosen from acrylic acid, methacrylic acid, ethylenic acid and sulphonic acid monomers, and the copolymers of these monomers with polymerizable monomers chosen from water-insoluble vinylaromatic monomers, such as styrene, methylstyrene, ethylstyrene, tert-butylstyrene or vinyltoluene, alone or as a mixture with other hydrophobic monomers.

28. The process as claimed in claims 14, 16, 26 or 27, **characterized in that** said weakly hydrophilic monomers are introduced into the phase A either simultaneously with the introduction of the hydrophobic monomers or before or after the introduction of said monomers.

29. The process as claimed in claim 14 or 16, **characterized in that** it additionally comprises, after the polymerization stage (iii), a stage of complete or partial evaporation of the phase A.

30. Conjugates, **characterized in that** they are composed of the composite particles as defined in claim 5, the functional groups of which have interacted, directly or indirectly, with at least one ligand chosen from an antibody, an antibody fragment, a protein, a polypeptide, an enzyme, a polynucleotide, a nucleic acid fragment and biotin.

31. A reagent, additionally comprising at least one conjugate as defined in claim 30.

32. Conjugates, **characterized in that** they are composed of the composite particles as defined in claim 5, the functional groups of which have interacted, directly or indirectly, with at least one ligand chosen from cage molecules, chelating agents and catalysts.

33. Conjugates, **characterized in that** they are composed of the composite particles as defined in claim 5, the functional groups of which have interacted, directly or indirectly, with at least one ligand chosen from medicinal substances, antisense probes, gene repair agents, or agents for blocking or inhibiting a protein activity.

34. Use of the conjugate as defined in claim 33 in a therapeutic composition.
